# EUROPEAN PATENT APPLICATION

(11) **EP 1 927 347 A1**
(43) Date of publication of application: **04.06.2008**
(21) Application number: 06782460.7
(22) Date of filing: 08.08.2006
(51) Int. Cl.: A61K 9/14, A61J 3/06, A61K 9/20, A61K 47/12, A61K 47/32, A61K 47/38

(54) **BITTERNESS-REDUCING AGENT**

(30) Priority: 10.08.2005 JP 2005232083
(71) Applicant: Shionogi Co., Ltd., Osaka-shi, Osaka 541-0045 (JP)
(72) Inventor: TANIGUCHI, Yoichi, Amagasaki-shi, Hyogo, 6600813 (JP); TOMODA, Yoshitaka, Settsu-shi, Osaka, 5660022 (JP); YOSHIOKA, Kazuaki, Amagasaki-shi, Hyogo, 6600813 (JP); TOYODA, Toshitada, Amagasaki-shi, Hyogo, 6600813 (JP); IMAMOTO, Chieko, Amagasaki-shi, Hyogo, 6600813 (JP)
(74) Representative: HOFFMANN EITLE
(86) International application number: PCT/JP2006/315618
(87) International publication number: WO 2007/018190

(57) **Abstract**

A powder-particle dosage form obtained by granulating an excipient and/or a disintegrating agent with a liquid in which a bitter taste masking base is dissolved and/or suspended, and a drug having solubility in water (20°C) of not higher than 1 g/mL is dissolved or suspended, or a tablet obtained by compressing the powder-particle dosage form could mask bitter taste of a drug.

## Description

### Technical Field

The present invention relates to a bitter taste masking dosage form, more particularly, a dosage form for masking bitter taste, obtained by granulating a highly viscous liquid in which a drug is dissolved or suspended.

### Background Art

As a dosage form which masks bitter taste, various preparations have been developed. More particularly, there is a dosage form obtained by adding a bitter substance to an ethanol solution with ethylcellulose which is a hydrophobic polymer and hydroxypropylcellulose which is a water-soluble polymer dissolved therein, and mixing the solution with an excipient (Patent Publication 1). In addition, even when a hydrophobic substance is not used, there is a fine granule in which a granule produced by spraying an ethanol solution of a gastrosoluble coating agent to a powder obtained by mixing a carbapenem antibody with lactose, is further mixed with lactose, polyvinylpyrrolidone or the like, and an aqueous hydroxypropylcellulose solution is sprayed to mask bitter taste (Patent Publication 2).
Patent Publication 1:
Japanese Patent Application Laid-Open (JP-A) No. 2002-363066 Patent Publication 2:
   JP-A No. 2004-35518

### Disclosure of Invention

### Problems to be solved by the Invention

However, in the case of a bitter taste masking dosage form of Patent Publication 1 using a hydrophobic polymer as a base, bitter taste is masked, but there is a high possibility that a dissolving rate of drug is suppressed. When a dissolving rate is suppressed, dissolution profile also varies, and there is a possibility that pharmacokinetics also varies. In addition, in both of Patent Publications 1 and 2, an organic solvent such as ethanol and the like is used in order to dissolve a base which masks bitter taste. However, in this case, it is necessary to additionally dispose an organic solvent recovering instrument in a machine, and the environment is adversely influenced.

### Means to Solve the Problems

Then, in order to solve the problems, the present inventors intensively studied and, as a result, a powder-particle dosage form (e.g. powders, fine granules, granules, syrups) obtained by dissolving or suspending a drug having solubility in water of not higher than 1 g/mL in a liquid having a high viscosity, adding the resulting liquid to an excipient and/or a disintegrating agent to granulate this masks bitter taste, resulting in completion of the present invention.

That is, the present invention relates to:
(1) a process for producing a powder-particle dosage form characterized in comprising the following steps:
   i) a step of dissolving or suspending a drug having solubility in water of not higher than 1 g/mL (20°C) in a liquid having a viscosity (20°C) having a viscosity of 50 to 14000 mPa·s,
   ii) a step of adding the solution or the suspension obtained in the step i) to an excipient and/or a disintegrating agent, and granulating this,
(2) the process for producing a powder/particle dosage form according to (1), wherein a viscosity of the liquid (20°C) is 500 to 14000 mPa·s,
(3) the process for producing a powder-particle dosage form according to (1) or (2), wherein the liquid contains hydroxypropylmethylcellulose,
(4) the process for producing a powder·particle dosage form according to (1) or (2), wherein the liquid contains fumaric acid, stearic acid and/or polyvinyl acetal diethyl aminoacetate,
(5) the process for producing a powder-particle dosage form according to (1), wherein the drug is a drug having solubility in water (20°C) of not higher than 0.1 g/mL,
(6) the process for producing a powder-particle dosage form according to (5), wherein the drug is a drug having solubility in water (20°C) of not higher than 0.033 g/mL,
(7) the process for producing a powder-particle dosage form according to any one of (1) to (6), wherein the excipient is a water-insoluble excipient,
(8) the process for producing a powder-particle dosage form according to any one of (1) to (7), wherein the granulation step is a wet granulation process,
(9) the process for producing a powder-particle dosage form according to (8), wherein the wet granulation process is any of a fluidized bed granulation process, a stirring granulation process, an extrusion granulation process, and a rolling granulation process,
(10) the process for producing a powder-particle dosage form according to (9), wherein the wet granulation process is a stirring granulation process,
(11) a powder-particle dosage form obtained by the process as defined in any one of (1) to (10),
(12) the powder-particle dosage form according to (11), which is a granule,
(13) the powder-particle dosage form according to (11) or (12), wherein bitter taste is masked,
(14) a process for producing a tablet, comprising compressing the powder-particle dosage form as defined in (11),
(15) a process for producing a tablet, comprising mixing the powder-particle dosage form as defined in (11) with an excipient, a lubricant, a disintegrating agent and/or a binder, and compressing the mixture,
(16) a tablet obtained by the process as defined in (14) or (15),
(17) the tablet according to (16), which is an orally disintegrating tablet,
(18) the tablet according to (16) or (17), wherein bitter taste is masked.

### Effect of the Invention

The bitter taste masking dosage form of the present invention attains bitter taste masking of drug by dissolving or suspending a drug having solubility of not higher than 1 g/mL which is a bitter taste component, in an additive solution having a high viscosity, adding the resulting liquid to an excipient and/or a disintegrating agent, and granulating this. The bitter taste masking dosage form of the present invention can mask bitter taste without suppressing dissolution.

### Best Mode for Carrying Out the Invention

A liquid for dissolving or suspending a drug may be such that a viscosity of a liquid at 20°C is adjusted at 50 to 14000 mPa·s, preferably 250 to 14000 mPa·s, more preferably 500 to 14000 mPa·s. When a viscosity is lower than this viscosity, there is a possibility that the bitter taste masking effect is not sufficiently exerted and, when a viscosity is higher than this viscosity, there is a possibility that an additive-blended liquid can not be dispersed in a granulator. It is necessary that this additive is dissolved and/or suspended in water. In the present description, "suspension" means that solid particles are observed in the liquid as colloidal particles or particles which can be observed by a microscope.

As for the liquid for dissolving or suspending a drug, it is necessary that an additive is added to water, and a viscosity is adjusted. An additive (hereinafter, referred to as "bitter taste masking base" in some cases) is a substance which is dissolved and/or suspended in water to be a liquid having a high viscosity, particularly a viscosity at 20°C of 50 to 14000 mPa·s, and which can be pharmaceutically used. Specifically, there are hydroxypropylmethylcellulose, fumaric acid·stearic acid·polyvinyl acetal diethyl aminoacetate·hydroxypropylmethylcellulose mixture, hydroxypropylcellulose, methylcellulose, hydroxyethylcellulose, sodium carmellose, polyvinylpyrrolidone K25, polyvinylpyrrolidone K30, polyvinylpyrrolidone K90, polyvinyl alcohol (complete saponified substance), polyvinyl alcohol (partial saponified substance), gum arabic, gum arabic powder, sodium alginate, alginic acid propylene glycol ester, agar, agar powder, gelatin, purified gelatin, tragacanth, xanthan gum, pregelatinized starch, partially pregelatinized starch, sodium carboxymethylstarch, pullulan, dextrin and the like. Preferable are hydroxypropylmethylcellulose, fumaric acid·stearic acid·polyvinyl acetal diethyl aminoacetate·hydroxypropylmethylcellulose 2910 mixture, hydroxypropylcellulose, polyvinylpyrrolidone K25, polyvinylpyrrolidone K30, polyvinylpyrrolidone K90 and the like. More preferable are hydroxypropylmethylcellulose, and fumaric acid·stearic acid·polyvinyl acetal diethyl aminoacetate· hydroxypropylmethylcellulose mixture. When an additive is "dissolved and suspended", it refers to dissolution of a part of the additive in water, and suspension of other part in water. For example, in the case of fumaric acid-stearic acid·polyvinyl acetal diethyl aminoacetate· hydroxypropylmethylcellulose mixture, fumaric acid·stearic acid·polyvinyl acetal diethyl aminoacetate are suspended in water, and hydroxypropylmethylcellulose is dissolved in water.

In the present invention, as hydroxypropylmethylcellulose which is a more preferable bitter taste masking base, any of these can be used as far as they can be pharmaceutically used. Specifically, there are hydroxypropylmethylcellulose 2208, hydroxypropylmethylcellulose 2906, and hydroxypropylmethylcellulose 2910 listed in Japanese pharmacopoeia. Particularly preferable is hydroxypropylmethylcellulose 2910. Hydroxypropylmethylcellulose 2910 is a mixed ester of methyl and hydroxypropyl of cellulose and, when quantitated at drying, a methoxyl group is contained at 28.0 to 30.0%, and a hydroxypropoxyl group is contained at 7.0 to 12.0%. As hydroxypropylmethylcellulose 2910, more specifically, there are TC-5E (manufactured by Shin-Etsu Chemical Co., Ltd.), Methocell E (Dow Chemical Japan), and Marpollose (Matsumoto Yushi-Seiyaku Co., Ltd.).

In the present invention, as a fumaric acid·stearic acid·polyvinyl acetal diethyl aminoacetate·hydroxypropylmethylcellulose mixture which is a more preferable bitter taste masking base, the mixture which can be pharmaceutically used may be used. Specifically, a fumaric acid-stearic acid-polyvinyl acetal diethyl aminoacetate·hydroxypropylmethylcellulose 2910 mixture described in Medicament Additive Specification 2003 (YAKUJI NIPPO LIMITED), more specifically, HA"Sankyo" (manufactured by Sankyo Company) can be used.

Particularly, in the case of hydroxypropylmethylcellulose which is an optimal additive in the present invention, specifically, when TC-5E (manufactured by Shin-Etsu Chemical Co., Ltd.) which is one kind of hydroxypropylmethylcellulose 2910 is used, a blending amount in liquid (hereinafter "weight %" is described as "w/w%" in some cases) is 6 to 35 w/w%, preferably 6.5 to 32.5 w/w%, more preferably 7 to 30 w/w%.

In addition, in the case of fumaric acid·stearic acid·polyvinyl acetal diethyl aminoacetate·hydroxypropylmethylcellulose mixture which is an optimal additive in the present invention, specifically, when HA"Sankyo" (manufactured by Sankyo Company) is used, a blending amount in liquid is 1 to 20 w/w%, preferably 2.5 to 15 w/w%, more preferably 5 to 10 w/w%.

A weight of hydroxypropylmethylcellulose of the present invention relative to a total amount of dosage form is 0.001 to 50 w/w%, preferably 0.01 to 30 w/w%, more preferably 0.1 to 25 w/w%. When a weight is smaller than this blending amount, there is a possibility that the bitter taste masking effect can not be sufficiently exerted and, when a weight is more than this, there is possibility that a dosage form is disintegration-delayed.

A weight of the fumaric acid·stearic acid·polyvinyl acetal diethyl aminoacetate·hydroxypropylmethylcellulose mixture of the present invention relative to a total weight of the dosage form is 0.001 to 60 w/w%, preferably 0.01 to 40 w/w%, more preferably 0.1 to 30 w/w%. When the weight is smaller than this blending amount, there is a possibility that the bitter taste masking effect can not be sufficiently exerted and, when a weight is more than this, there is possibility that a dosage form is disintegration-delayed.

The drug used in the present invention has solubility in water at 20°C of not higher than 1 g/mL, preferably not higher than 0.1 g/mL, more preferably not higher than 0.033 g/mL, and it is enough that the drug is dissolved or suspended in water. When solubility in water is higher than these solubilities, there is a possibility that it becomes difficult to mask bitter taste.

As the drug used in the present invention, one or two or more kinds of components selected from a nutritional health supplement, an antipyretic-analgesic-antiphlogistic, a psychotropic, an anti-anxiety agent, an anti-depressant, a hypnotic-sedative, an antispasmodic, a central nerve-acting drug, a brain metabolism improving agent, a brain circulation improving agent, an antiepileptic, a sympathomimetic agent, a medicine for digestive system, an antacid, an anti-ulcer agent, an antitussive-expectorant, an antiemetic, a respiratory promoter, a bronchodilator, a drug for allergy, a dental oral drug, an anti-histamine agent, a cardiotonic agent, a drug for arrhythmia, a diuretic, an antihypertensive, a vasoconstrictor, a coronary vasodilator, a peripheral vasodilator, a drug for hyperlipemia, a cholagogue, an antibiotic, a chemotherapeutic, a drug for diabetes, a drug for osteoporosis, an antirheumatic, a skeletal muscle relaxant, an antispasmodic, a hormone agent, an alkaloid narcotic, a sulfa drug, a gout treating drug, a blood coagulation preventing agent, and an anti-malignant tumor agent are used.

Examples of the nutritional health supplement include vitamins such as vitamin A, vitamin D, vitamin E (d- α -tocopherol acetate etc.), vitamin B1 (dibenzoylthiamine, fursultiamine hydrochloride etc.), vitamin B2 (riboflavine butyrate etc.), vitamin B6 (pyridoxine hydrochloride etc.), vitamin C (ascorbic acid, sodium L-ascorbate etc.), and vitamin B12 (hydroxocobalamine acetate, cyanocobalamine etc.), minerals such as calcium, magnesium and iron, proteins, amino-acids, oligosaccharides, and crude drugs. Examples of the antipyretic-analgesic-antiphlogistic include aspirin, acetoaminophen, ethenzamide, eveprophen, diphenhydramine hydrochloride, chlorpheniramine dl-maleate, dihydrocodeine phosphate, noscapine, methylephedrine hydrochloride, phenylpropanolamine hydrochloride, caffeine, anhydrous caffeine, serrapeptase, lysozyme chloride, tolfenamic acid, mefenamic acid, diclofenac sodium, flufenamic acid, salicylamide, aminopyrine, ketoprophen, indometacin, bucolome, and pentazocine.

Examples of the psychtropic include chlorpromazine, and reserpine. Examples of the anti-anxiety drug include alprazolam, chlordiazepoxide, and diazepam. Examples of the anti-depressant include imipramine, maprotiline hydrochloride, and amphetamine. Examples of the hypnotic-sedative include estazolam, nitrazepam, diaaepam, perlapine, and phenobarbital sodium. Examples of the antispasmodic include scopolamine hydrobromide, diphenhydramine hydrochloride, and papaverine hydrochloride. Examples of the central nerve-acting drug include citicoline. Examples of the brain metabolism improving agent include meclofenoxate hydrochloride. Examples of the brain circulation improving agent include vinpocetine. Examples of the antiepileptic include phenytoin and carbamazepine. Examples of the sympathomimetic agent include isoproterenol hydrochloride. Examples of the medicine for digestive system include stomachic digestants such as diastase, sugar-containing pepsin, scopolia extract, cellulase AP3, lipase AP, and cinnamon oil, and medicine for intestinal disorders such as berberine chloride, resistant lactic acid bacterium, and Bifidobacteria.

Examples of the antacid include magnesium carbonate, sodium bicarbonate, magnesium aluminate metasilicate, synthetic hydrotalcite, precipitated calcium carbonate, and magnesium oxide. Examples of the anti-ulcer agent include lansoprazole, omeprazole, rabeprazole, famotidine, cimetidine, and ranitidine hydrochloride. Examples of the antitussive-expectorant include cloperastine hydrochloride, dextromethorphan hydrobromide, theophylline, potassium guacacolsulfonate, guaifenesin, and codeine phosphate. Examples of the antiemetic include difenidol hydrochloride, and metoclopramide. Examples of the respiratory promoter include levallorphan tartrate. Examples of the bronchodilator include theophylline, and salbutamol sulfate. Examples of the drug for the allergy include amlexanox, and seratrodast. Examples of the dental oral drug include oxytetracycline, triamcinolone acetomide, chlorhexidine hydrochloride, and lidocaine.

Examples of the anti-histamine agent include diphenhydramine hydrochloride, promethazine, isothipendyl hydrochloride, and chlorpheniramine dl-maleate. Examples of the cardiotonic agent include caffeine, and digoxin. Examples of the agent for arrhythmia include procaineamide hydrochloride, propranolol hydrochloride, and pindolol. Examples of the diuretic include isosorbide, furosemide, and hydrochlorothiazide. Examples of the antihypertensive include delapril hydrochloride, captopril, hydralazine hydrochloride, labetalol hydrochloride, manidipine hydrochloride, candesartan cilexetil, methyldopa, and perindopril erbumine. Examples of the vasoconstrictor include phenylephrine hydrochloride.

Examples of the coronary vasodilator include carbocromen hydrochloride, molsidomine, and verapamil hydrochloride. Examples of the peripheral vasodilator include cinnarizine. Examples of the agent for hyperlipemia include cerivastatin sodium, simvastatin, pravastatin sodium, and atorvastatin calcium hydrate. Examples of the cholagogue include dehydrocholic acid, and trepibutone. Examples of the antibiotic include cephem antibiotics such as cephalexin, cefaclor, amoxicillin, pivmecillinam hydrochloride, cefotiam hexetil hydrochloride, cefadroxil, cefixime, cefditoren pivoxil, cefteram pivoxil, and cefpodoxymiproxetil, monobactam, penem and carbapenem antibiotics such as ampicillin, ciclacillin, nalidixic acid, and synthetic anti-fungal agents such as enoxacin, and carumonam sodium.

Examples of the chemotherapeutic include sufamethizol. Examples of the agent for diabetes include tolbutamide, voglibose, pioglitazone hydrochloride, glibenclamide, and troglitazon. Examples of the agent for osteoporosis include ipriflavone. Examples of the skeletal muscle relaxant include methocarbamol. Examples of the antispasmodic include meclizine hydrochloride, and dimenhydrinate. Examples of the antirheumatic include methotrexate, and bucillamine. Examples of the hormone agent include liothyronine sodium, dexamethasone phosphate sodium, prednisolone, oxendolone, and leuprorelin acetate. Examples of the alkaloid narcotic include opium, morphine hydrochloride, ipecac, oxycodone hydrochloride, opium alkaloid hydrochloride, and cocaine hydrochloride. Examples of the sulfa drug include sulfisomidine, and sufamethizol. Examples of the gout treating drug include allopurinol, and colchicine. Examples of the blood coagulation preventing agent include dicumarol. Examples of the anti-malignant tumor agent include 5-fluorouracil, uracil, and mitomycin.

These drugs can be used alone, or as a combined agent with another drug. In addition, these drugs are administered at the known suitable amount which is conveniently determined depending on a disease an age and the like of a patient.

A drug blending amount of hydroxypropylmethylcellulose or the fumaric acid·stearic acid·polyvinyl acetal diethyl aminoacetate· hydroxypropylmethylcellulose mixture of the present invention in a liquid is 0.001 to 40 w/w%, preferably 1 to 30 w/w%, more preferably 2 to 20 w/w%. When an amount is larger than this blending amount, there is a possibility that bitter taste of the drug can not be masked.

A blending ratio of hydroxypropylmethylcellulose or the fumaric acid·stearic acid·polyvinyl acetal diethyl aminoacetate· hydroxypropylmethylcellulose mixture and the drug is preferably 1000:1 to 0.5:1, preferably 100:1 to 0.75:1, more preferably 10:1 to 1:1. When a blending ratio of hydroxypropylmethylcellulose or the fumaric acid·stearic acid·polyvinyl acetal diethyl aminoacetate·hydroxypropylmethylcellulose mixture and the drug is more than 1000:1, there is a possibility that dissolution of the drug is delayed. On the other hand, when a ratio of the drug is more than a blending ratio of hydroxypropylmethylcellulose or fumaric acid·stearic acid·polyvinyl acetal diethyl aminoacetate· hydroxypropylmethylcellulose mixture and the drug 0.5:1, there is a possibility that bitter taste of the drug can not be masked.

A method of preparing a solution or a suspension of the drug is not particularly limited: for example, there are a method of dissolving or suspending a predetermined amount of a drug in a liquid in which a predetermined amount of hydroxypropylmethylcellulose or the fumaric acid·stearic acid·polyvinyl acetal diethyl aminoacetate· hydroxypropylmethylcellulose mixture is dissolved and/or suspended in water, and a method of dissolving or suspending a predetermined amount of the drug in water, and dissolving and/or suspending hydroxypropylmethylcellulose or the fumaric acid·stearic acid·polyvinyl acetal diethyl aminoacetate·hydroxypropylmethylcellulose mixture in the liquid.

In the present invention, an excipient used is enough that it can be pharmaceutically used. Specifically, any of a water-soluble excipient and a water-insoluble excipient can be used. Examples of more specific excipient include glucose, fructose, lactose, sucrose, D-mannitol, erythritol, maltitol, trehalose, sorbitol, corn starch, potato starch, wheat starch, rise starch, crystalline cellulose, anhydrous silicic acid, anhydrous dibasic calcium phosphate, calcium hydrogen phosphate, calcium carbonate, precipitated calcium carbonate, calcium silicate, hydrous titanium dioxide and the like. Preferable are water-insoluble excipients such as crystalline cellulose, calcium carbonate, precipitated calcium carbonate, anhydrous dibasic calcium phosphate, calcium hydrogen phosphate and the like.

A blending amount of the excipient of the present invention is 0 to 99.9 w/w%, preferably 5 to 90 w/w%, more preferably 30 to 70 w/w%, relative to a total amount of the dosage form. When an amount is less than this blending amount, there is a possibility that a powder·particle dosage form can not be formed.

In the present invention, a disintegrating agent may be used. Particularly, in the case of stirring granulation, a disintegrating agent is required. As the disintegrating agent used, any disintegrating agent can be used as far as it can be pharmaceutically used. Specifically, examples of the disintegrating agent include low-substituted hydroxypropylcellulose, carmellose, carmellose calcium, carboxymethylstarch sodium, croscarmellose sodium, crospovidone, corn starch, pregelatinized starch, agar powder and the like. Preferable is crospovidone.

A blending amount of the disintegrating agent of the present invention is 0 to 99.9 w/w%, preferably 5 to 90 w/w%, more preferably 10 to 70 w/w% based on a total amount of the dosage form. When an amount is smaller than this blending amount, there is a possibility that the disintegrating property is reduced.

In the present invention, a binder may be used. As the binder used, any binder can be used as far as it can be pharmaceutically used. Specifically, examples include hydroxypropylmethylcellulose, fumaric acid· stearic acid·polyvinyl acetal diethyl aminoacetate· hydroxypropylmethylcellulose mixture, hydroxypropylcellulose, carmellose, carmellose sodium, carboxymethylstarch sodium, croscarmellose sodium, crospovidone, hydroxyethylmethylcellulose, hydroxypropylstarch, hydroxyethylcellulose and the like. Preferable are hydroxypropylmethylcellulose or fumaric acid·stearic acid·polyvinyl acetal diethyl aminoacetate·hydroxypropylmethylcellulose mixture.

A blending amount of the binder of the present invention is 0 to 20 w/w% relative to a total amount of the dosage form. When an amount is larger than this blending amount, there is a possibility that disintegration is delayed.

In the present invention, a corrigent may be used in order to alleviate bitter taste. Specifically, there are a mint oil, and citric acid.

In the present invention, a blending amount of hydroxypropylmethylcellulose which is one of optimal bitter taste masking bases, and the drug in a liquid, when TC-5E (manufactured by Shin-Etsu Chemical Co., Ltd.) which is one kind of hydroxypropylmethylcellulose 2910 is used, is such that hydroxypropylmethylcellulose is 6 to 35 w/w%, the drug is 0.001 to 40 w/w%, preferably hydroxypropylmethylcellulose is 6.5 to 32.5 w/w%, and the drug is 1 to 30 w/w%, more preferably hydroxypropylmethylcellulose is 7 to 30 w/w%, and the drug is 2 to 20 w/w%.

In the present invention, a blending amount of fumaric acid·stearic acid·polyvinyl acetal diethyl aminoacetate·hydroxypropylmethylcellulose mixture which is one of optimal bitter taste masking bases, and the drug in a liquid, when HA "Sankyo"(manufactured by Sankyo Company) which is fumaric acid·stearic acid·polyvinyl acetal diethyl aminoacetate· hydroxypropylmethylcellulose 2910 mixture is used, is such that fumaric acid·stearic acid·polyvinyl acetal diethyl aminoacetate· hydroxypropylmethylcellulose 2910 mixture is 1 to 20 w/w%, and the drug is 0.001 to 40 w/W%, preferably the mixture is 2.5 to 15 w/w%, and the drug is 1 to 30 w/w%, more preferably the mixture is 5 to 10 w/w%, and the drug is 2 to 20 w/w%.

The powder-particle dosage form of the present invention is specifically powders, granules and syrups in General Rules for Preparations described in Japanese Pharmacopoeia 14^{th} revision. Particularly, the process of the present invention is a process which is optimal for producing granules, particularly granules whose bitter taste is masked.

As the process for producing a powder-particles dosage form in the present invention, any process can be used as far as it can be pharmaceutically used, and there are a wet granulation process, preferably a fluidized bed granulation process, a stirring granulation process or an extrusion granulation process, and a rolling granulation process, more preferably a stirring granulation process. The fluidized bed granulation process is a method of spraying a binder to a powder fluidized in a bed to obtain a granulated product. The stirring granulation process is a method of adding a binder to a subject substance charged in a tank, and imparting shearing·rolling·compacting actions by rotating a stirring wing having a variety shapes to obtain an objective granulated product. The extrusion granulation process is a method of forcibly extruding a kneaded wet mass through a screen having a suitable size to obtain a granulated product. And, the rolling granulation process is a method of rolling a raw material powder moved towards an external wall part with a centrifugal force of a rotating rotor, with the air blown up from a slit, and spraying a binder thereupon to obtain a granulated product. In addition, in a spraying drying method, a production time becomes longer than that of the above processes, and it is difficult to control bitter taste of the drug.

When the dosage form of the present invention is produced by the fluidized bed granulation process, a solution and/or a suspension of a bitter taste masking base in which the drug is dissolved or suspended in advance is prepared, and the aforementioned liquid is sprayed, and granulation is performed while an excipient and/or a disintegrating agent is flown in a fluidized bed.

When the dosage form of the present invention is produced by the stirring granulation process, a solution and/or a suspension of a bitter taste masking base in which the drug is dissolved or suspended in advance is prepared, and the aforementioned liquid is added, granulation is performed while an excipient and/or a disintegrating agent is stirred in a tank of a stirring granulator.

When the dosage form of the present invention is produced by an extrusion granulation process, a solution and/or a suspension of a bitter taste masking base in which the drug is dissolved or suspended in advance is prepared, the liquid is mixed and kneaded with an excipient and/or a disintegrating agent, and this kneaded product is extruded, and granulation is performed with a extrusion granulator.

When the dosage form of the present invention is produced by the rolling granulation process, a solution and/or a suspension of a bitter taste masking base in which the drug is dissolved or suspended in advance is prepared, and the aforementioned liquid is added, and granulation is performed while an excipient and/or a disintegrating agent is rolled in a tank of a centrifugation rolling granulating apparatus.

An excipient, a disintegrating agent, a binder and a lubricant together with the powder·particle dosage form of the present invention may be mixed, and compressed to form a tablet. As the excipient, the disintegrating agent and the binder, the aforementioned substances can be used. In addition, as the lubricant, there are magnesium stearate, sucrose fatty acid ester, magnesium carbonate and the like.

Alternatively, a tablet can be also produced by an external lubricating compressing method of mixing an excipient, a disintegrating agent and a binder together with the powder-particle dosage form of the present invention, attaching a small amount of a lubricant to a mortar and a mallet of a compressing machine, and compressing the mixture. In the compressing method, compressing can be performed with a small amount of a lubricant, and this is useful compressing method for a drug which is easily denatured with a lubricant.

A new type tablet which is instantly disintegrated in an oral cavity, and can mask bitter taste can be produced by blending the powder·particle dosage form of the present invention into an oral disintegrating tablet which is instantly disintegrated in an oral cavity besides the usual tablets. In the case of the oral disintegrating tablet, as the excipient, the disintegrating agent, the binder and the lubricant, the aforementioned general additives can be used. In addition, in order to render ingestion feeling good, a corrigent such as a mint oil and citric acid can be also added to a tablet.

### Examples

The present invention will be explained in more detail below by way of Examples, but Examples are merely exemplification, and do not limit the present invention.

### 1. Selection of bitter taste masking base

### (Experimental method)

In order to screen a bitter taste masking base used in bitter taste masking, the bitter taste masking effect by a cast film was confirmed. After dosage form of a liquid shown in Table 1, a cast film was prepared as follows. A bitter taste masking base and purified water are placed into a mold mug to dissolve and/or suspend the base, and a predetermined amount of a bitter taste masking liquid is weighed. To this liquid is added a drug to uniformly suspend the drug, and a dispersion is spread thin on a weighing dish. This is air-dried with a ventilation dryer to prepare a cast film. The drying condition with a ventilation dryer is a ventilation temperature of 60°C and a drying time of 1 hour. For accessing bitter taste, the above-prepared cast film is contained in an oral cavity of 5 healthy adults, and an organoleptic test was performed on a tongue for about 5 seconds.
A ratio of blending a drug and a bitter taste masking base was 1:1 and 1:3 as expressed by a weight ratio (solid matter). As the drug, isopropylantipyrine (manufactured by KONGO CHEMICAL CO., LTD.) was used. As the bitter taste masking base, hydroxypropylcellulose (HPCSL, manufactured by Nippon Soda Co., Ltd.), hydroxypropylmethylcellulose 2910 (TC-5E, manufactured by Shin-Etsu Chemical Co., Ltd.), fumaric acid-stearic acid·polyvinyl acetal diethyl aminoacetate· hydroxypropylmethylcellulose 2910 mixture (HA Sankyo, manufactured by Sankyo Company), and polyvinylpyrrolidone K25 (Povidone, manufactured by BASF Japan Ltd.) were used. Table 1 showed bitter taste masking bases used, and Table 2 showed a composition of a bitter taste masking dosage form. In addition, a viscosity of a bitter taste masking base liquid at 20°C described in Table 2 before preparation of a cast film was 50 to 14000 mPa·s in all cases.

**[Table 1]**

| Bitter taste masking base | |
|---|---|
| General name | Abbreviation |
| Hydroxypropylcellulose | HPC |
| Hydroxypropylmethylcellulose 2910 | HPMC |
| Fumaric acid·stearic acid-polyvinyl acetal diethyl aminoacetate· hydroxypropylmethylcellulose 2910 mixture | HA-Sankyo |
| Polyvinylpyrrolidone K25 | PVP |

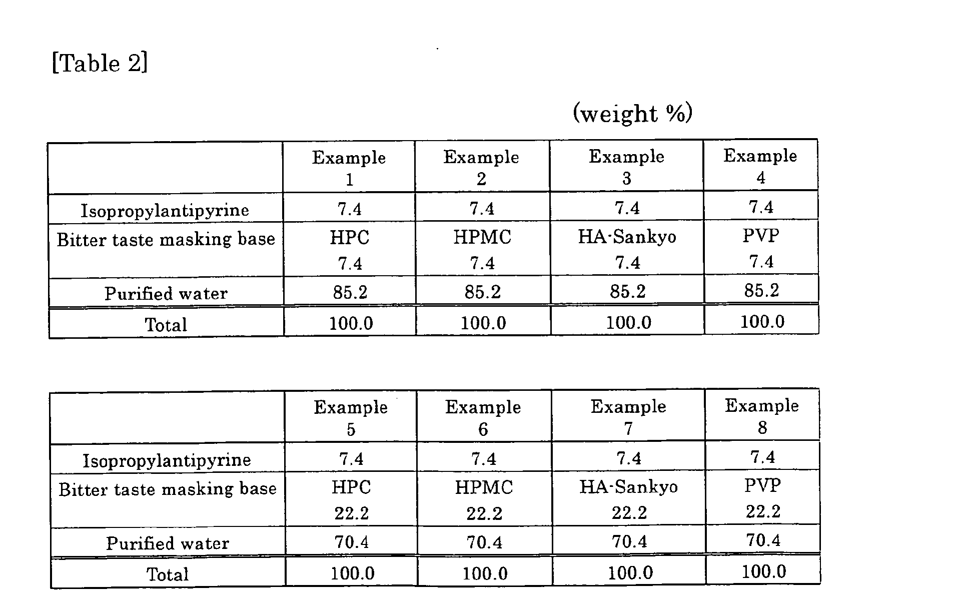

### (Experimental result)

Results are shown in Table 3 and Table 4. As a result, the masking effect was recognized in all bases. Inter alia, HPMC and HA Sankyo had the highest effect.

**[Table 3]**

| Ratio of blending drug: bitter taste masking base = 1:1(ratio by weight) | | | | |
|---|---|---|---|---|
| | Example 1 | Example 2 | Example 3 | Example 4 |
| Bitter taste masking base | HPC | HPMC | HA-Sankyo | PVP |
| Masking effect | ○ | ○ | ⊚ | ○ |

| | | | | |
|---|---|---|---|---|
| ⊚: Masking effect is very high. ○: Masking effect is high. Δ: Slight masking effect. ×: No masking effect. | | | | |

**[Table 4]**

| Ratio of blending drug: bitter taste masking base = 1:3(ratio by weight) | | | | |
|---|---|---|---|---|
| | Example 5 | Example 6 | Example 7 | Example 8 |
| Bitter taste masking base | HPC | HPMC | HA-Sankyo | PVP |
| Masking effect | ○ | ⊚ | ⊚ | ○ |

| | | | | |
|---|---|---|---|---|
| ⊚: Masking effect is very high. ○: Masking effect is high. Δ: Slight masking effect. ×: No masking effect. | | | | |

### 2. Selection of liquid viscosity

### (Experimental method)

Preparation of the cast film is as described in the (1) Experimental method. As a formulation liquid, a liquid in Table 5 was prepared. A ratio of blending a drug and a bitter taste masking base was 1:3 as expressed by a weight ratio (solid matter). As a viscosity, a viscosity of a liquid in which hydroxypropylmethylcellulose [HPMC] was dissolved in purified water was measured. A method of measuring a viscosity was performed based on ""Method II: Viscosity measurement by rotational viscometer" in section "Viscosity determination" of Japanese Pharmacopoeia 14^{th} revision. As the viscometer, a digital viscometer (manufactured by Brookfield, Model DV-II+, Spindle LV2) was used, and measurement was performed at 20°C. As the drug, isopropylantipyrine (manufactured by KONGO CHEMICAL CO., LTD.) was used. In addition, as hydroxypropylmethylcellulose [HPMC] which is a bitter taste masking base, hydroxypropylmethylcellulose 2910 [HPMC] (TC-5E, manufactured by Shin-Etsu Chemical Co., Ltd.) was used.

**[Table 5]**

| (weight %) | | | | | | |
|---|---|---|---|---|---|---|
| | Comparative Example 1 | Example 9 | Example 10 | Example 11 | Example 12 | Comparative Example 2 |
| Isopropylantipyrine | 11.8 | 9.1 | 6.3 | 4.8 | 3.2 | 1.6 |
| HPMC | 35.2 | 27.3 | 18.9 | 14.3 | 9.7 | 4.9 |
| Purified water | 53.0 | 63.6 | 74.8 | 80.9 | 87.1 | 93.5 |
| Total | 100.0 | 100.0 | 100.0 | 100.0 | 100.0 | 100.0 |

### (Experimental result)

Results of a liquid viscosity and an organoleptic test are shown in Table 6. As a result, in Comparative Example 1, a viscosity was high (higher than 14000mPa·s), hydroxypropylmethylcellulose was not dissolved, and the drug could not be dissolved or suspended. On the other hand, in Comparative Example 2, a liquid viscosity became 15 mPa·s, and bitter taste could not be masked. When a viscosity was 500 to 14000 mPa·s, the masking effect became very high.

**[Table 6]**

| | Comparative Example 1 | Example 9 | Example 10 | Example 11 | Example 12 | Comparative Example 2 |
|---|---|---|---|---|---|---|
| Viscosity of HPMC liquid (mPa·s) | Not dissolved | 13317.0 | 710.8 | 120.0 | 64.2 | 15.0 |
| Masking effect | - | ⊚ | ⊚ | ○ | ○ | × |

| | | | | | | |
|---|---|---|---|---|---|---|
| ⊚: Masking effect is very high. ○: Masking effect is high. Δ: Slight masking effect. ×: No masking effect. | | | | | | |

### 3. Difference in bitter taste depending on solubility in water of drug

### (Experimental method)

Preparation of the cast film is as described in 1. As a formulation liquid, a liquid in Table 7 was prepared. As the drug, anhydrous caffeine, acetaminophen, rilmazafone hydrochloride and nicotinic acid amide were used. In addition, as hydroxypropylmethylcellulose which is a bitter taste masking base, hydroxypropylmethylcellulose 2910 [HPMC] (TC-5 E, manufactured by Shin-Etsu Chemical Co., Ltd.) was used. In addition, a ratio of blending a drug and a bitter taste masking base was 1:3 as expressed by a weight ratio (solid matter).
According to expression of General Notices of Japanese Pharmacopoeia14^{th} revision, solubility in water of the drugs is such that isopropylantipyrine is "hardly soluble " (not less than 0.001 g/mL and less than 0.01 g/mL), anhydrous caffeine is "slightly hardly soluble"(not less than 0.01 g/mL and less than 0.033 g/mL), acetaminophen is "slightly hardly soluble" (not less than 0.01 g/mL and less than 0.033 g/mL), rilmazafone hydrochloride is "slightly soluble" (not less than 0.033 g/mL and less than 0.1 g/mL), and nicotinic acid amide is "easily soluble " (not less than 0.1 g/mL and less than 1.0 g/mL). A viscosity of a HPMC solution at 20°C described in Table 7 is 50 - 14000 mPa·s.

**[Table 7]**

| (weight %) | | | | |
|---|---|---|---|---|
| | Example 13 | Example 14 | Example 15 | Example 16 |
| Drug | Anhydrous caffeine 6.3 | Acetaminophen 6.3 | Rilmazafone hydrochloride 6.3 | Nicotinic acid amide 6.3 |
| HPMC | 18.9 | 18.9 | 18.9 | 18.9 |
| Purified water | 74.8 | 74.8 | 74.8 | 74.8 |
| Total | 100.0 | 100.0 | 100.0 | 100.0 |

### (Experimental result)

Results of an organoleptic test are shown in Table 8. As a result, even when solubility in water of the drug was changed, the masking effect was not changed, and the masking effect was high in all drugs.

**[Table 8]**

| | Example 13 | Example 14 | Example 15 | Example 16 |
|---|---|---|---|---|
| Drug | Anhydrous caffeine | Acetaminophen | Rilmazafone hydrochloride | Nicotinic acid amide |
| Masking effect | ○ | ○ | ○ | ○ |

| | | | | |
|---|---|---|---|---|
| ⊚: Masking effect is very high. ○: Masking effect is high. Δ: Slight masking effect. ×: No masking effect. | | | | |

### 4. Comparison of difference in bitter taste by drug addition method

### (Experimental method)

Dosage form formulation is shown in Table 9. Anhydrous dibasic calcium phosphate and carmellose calcium were added to a high speed mixer (Model FS2, High Speed Mixer), a liquid in which a predetermined amount of a drug was suspended in a 20 w/w% aqueous solution of hydroxypropylmethylcellulose [HPMC] was separately added, and the mixture was stirred and granulated. The granulating product was dried with a fluidized bed granulator. A ratio of blending a drug and a bitter taste masking base was 1:3 as expressed by a weight ratio (solid matter). On the other hand, as comparative formulation, the drug, a disintegrating agent and an excipient were mixed, 20 w/w% hydroxypropylmethylcellulose [HPMC] was added thereto, and the mixture was stirred and granulated. As the drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used. As the excipient, anhydrous dibasic calcium phosphate (manufactured by Kyowa Chemical Industry Co., Ltd.) was used. As the disintegrating agent, carmellose calcium (manufactured by Gotoku Chemical Company LTD.) was used. In addition, a viscosity of a HPMC solution at 20°C described in Table 9 is 50 to 14000 mPa·s.

### Granulation conditions

· Granulator: Model FS2, High Speed Mixer
· Production scale: about 150 g
· Mixer rotation number:300 rpm
· Chopper rotation number:2500 rpm

### Drying conditions

· Dryer: Model FL-MINI fluidized bed granulator
· Ventilation temperature: 75°C
· Drying completion: Exhaust gas temperature 45°C

### Particle adjusting machine: Model P-3 Power Mill

· Rotation number: 3000 rpm
· Screen: 0.5 mm herringbone

**[Table 9]**

| (weight %) | | |
|---|---|---|
| | Example 17 | Comparative Example 3 |
| Drug addition method | A drug was suspended in a HPMC solution, and granulation was performed using the solution | A drug, a disintegrating agent and an excipient were mixed, and granulation was performed using a HPMC solution. |
| Isopropylantipyrine | 4.2 | 4.2 |
| HPMC | 12.5 | 12.5 |
| Disintegrating agent | Carmellose calcium 41.65 | Carmellose calcium 41.65 |
| Excipient | Anhydrous dibasic calcium phosphate 41.65 | Anhydrous dibasic calcium phosphate 41.65 |
| Total | 100.0 | 100.0 |

### (Experimental results)

Results of an organoleptic test are shown in Table 10. As a result, in the case of Example 17 where a drug was suspended in a HPMC solution, the liquid was sprayed to a disintegrating agent and an excipient and stirring granulation were performed, bitter taste could be masked and the masking effect was high. On the other hand, when a drug was not suspended in a HPMC solution, bitter taste could not be masked.

**[Table 10]**

| | Example 17 | Comparative Example 3 |
|---|---|---|
| Drug addition method | A drug was suspended in a·HPMC solution, and granulation was performed using the solution. | A drug, a disintegrating agent and an excipient were mixed, and granulation was performed using a HPMC solution. |
| Masking effect | ○ | × |

| | | |
|---|---|---|
| ⊚: Masking effect is very high. ○: Masking effect is high. Δ: Slight masking effect. ×: No masking effect. | | |

### 5. Comparison of process for producing granule

### (1) Fine particle coating method

### (Experimental method)

A drug was placed into a fluidized bed granulator (composite-type fluidized bed granulation coating apparatus, manufactured by Fuji Paudal co., ltd), and a 5 w/w% aqueous solution of hydroxypropylmethylcellulose [HPMC] was sprayed while flown. A ratio of blending a drug and a bitter taste masking base was 1:3 as expressed by a weight ratio (solid matter). As the drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used.

### (Experimental results)

As a result of an organoleptic test, bitter taste of the drug could not be masked.

### (2) Fluidized bed granulation process

### (Experimental method)

Dosage form formulation is shown in Table 11. The following excipient was placed into a fluidized bed granulator (Model WSG2 fluidized bed granulator, manufactured by OKAWARA MFG. CO., LTD.). Separately, a predetermined amount of a drug was suspended in a 10 w/w% aqueous solution of hydroxypropylmethylcellulose [HPMC]. A ratio of blending a drug and a bitter taste masking base was 1:3 as expressed by a weight ratio (solid matter). The hydroxypropylmethylcellulose solution in which the drug was suspended was sprayed to an excipient flowing in the fluidized bed granulator under the following conditions. As the drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used. As the excipient, anhydrous dibasic calcium phosphate (manufactured by Kyowa Chemical Industry Co., Ltd.), crystalline cellulose (manufactured by Asahi Kasei Corporation), and precipitated calcium carbonate (manufactured by Shiraishi Calcium Kaisha, Ltd.) were used. In addition, a viscosity of a HPMC solution at 20°C described in Table 11 is 50 to 14000 mPa·s.

### Granulation conditions

· Granulator : Model WSG2 fluidized bed granulator
· Production scale: 2 kg
· Spray nozzle diameter: 1.2 mm
· Spray pressure: 0.2 MPa
· Spray solution flow rate: 10 to 20 g/min
· Ventilation temperature: 80°C
· Product temperature: 50°C

**[Table 11]**

| (weight %) | | | |
|---|---|---|---|
| | Example 18 | Example 19 | Example 20 |
| Isopropylantipyrine | 4.2 | 4.2 | 4.2 |
| HPMC | 12.5 | 12.5 | 12.5 |
| Excipient | Anhydrous dibasic calcium phosphate 83.3 | Crystalline cellulose 83.3 | Precipitated calcium carbonate 83.3 |
| Total | 100.0 | 100.0 | 100.0 |

### (Experimental result)

Experimental results are shown in Table 12. As a result, bitter taste could be masked using any excipient.

**[Table 12]**

| | Example 18 | Example 19 | Example 20 |
|---|---|---|---|
| Excipient | Anhydrous dibasic calcium phosphate | Crystalline cellulose | Precipitated calcium carbonate |
| Masking effect | ○ | ○ | ○ |

| | | | |
|---|---|---|---|
| ⊚: Masking effect is very high. ○: Masking effect is high. Δ: Slight masking effect. ×: No masking effect. | | | |

### (3) Stirring granulation process

### (Experimental method)

Dosage form formulation is shown in Table 13. An excipient and a disintegrating agent described in Table 7 were placed into a high speed mixer (Model FS2 High Speed Mixer), a liquid in which a predetermined amount of a drug was suspended in a 20 w/w% aqueous solution of hydroxylpropylmethylcellulose [HPMC] was added separately, and the mixture was stirred and granulated. A ratio of blending a drug and a bitter taste masking base was 1:3 as expressed by a weight ratio (solid matter). A granule was dried with a fluidized bed granulator. As the drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used. As an excipient, anhydrous dibasic calcium phosphate (manufactured by Kyowa Chemical Industry Co., Ltd.) was used. As a disintegrating agent, carmellose calcium (manufactured by Gotoku Chemical Company LTD.), crospovidone (manufactured by ISP Japan), carmellose sodium (manufactured by Gotoku Chemical Company LTD.), low-substituted hydroxypropylcellulose (manufactured by Shin-Etsu Chemical Co., Ltd.), corn starch (manufactured by NIHON SHOKUHIN KAKO CO., LTD) and carmellose (manufactured by Gotoku Chemical Company LTD.) were used. In addition, a viscosity of a HPMC solution at 20°C described in Table 13 is 50 to 14000 mPa·s.

### Stirring granulation conditions

· Granulator: Model FS2 High Speed Mixer
· Production scale: about 150 g
· Mixer rotation number: 300 rpm
· Chopper rotation number: 2500 rpm

### Drying conditions

· Dryer: Model FL-MINI fluidized bed granulator
· Ventilation temperature: 75°C
· Drying completion: Exhaust air temperature 45°C

### Particle adjusting conditions

· Particle adjusting machine: Model P-3 Power Mill
· Rotation number: 3000 rpm
· Screen: 0.5 mm herringbone

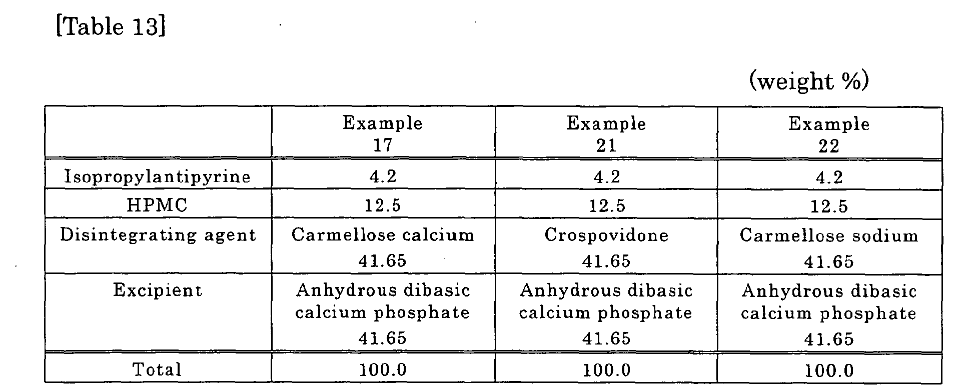

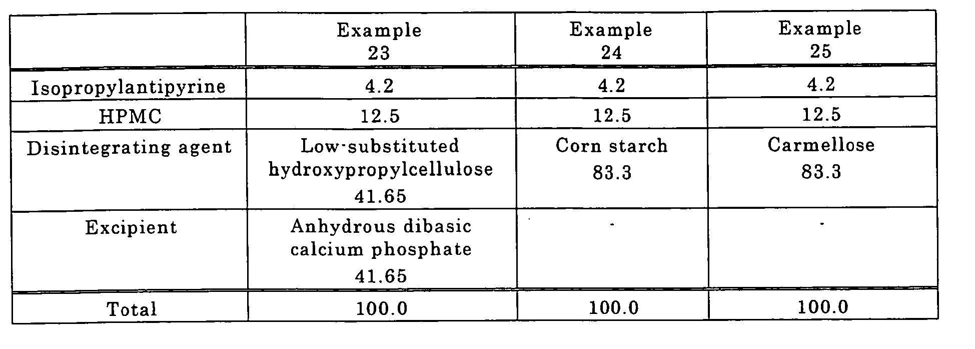

### (Experimental result)

Results of an organoleptic test are shown in Table 14. As a result, bitter taste could be masked using any excipient or this integrating agent. Particularly, when crospovidone was used as a disintegrating agent, bitter taste was particularly masked.

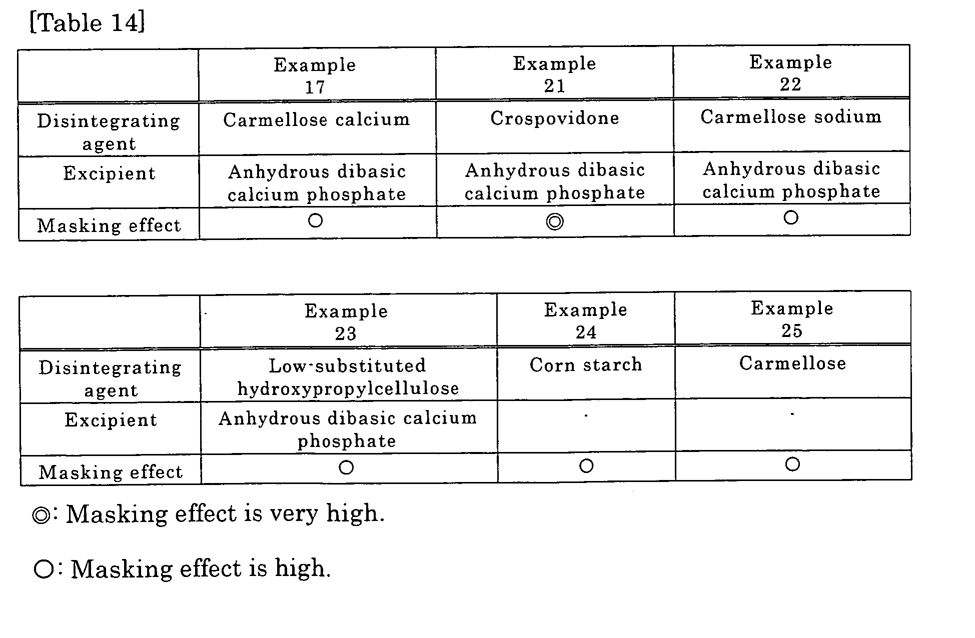

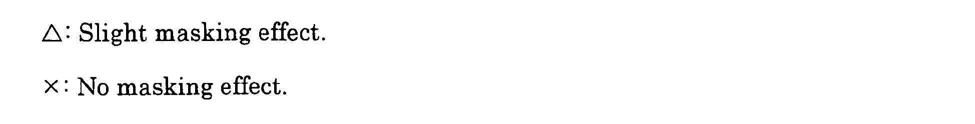

### (4) Rolling granulation process

### (Experimental method)

Dosage form formulation is shown in Table 15. The following excipient was placed into a rolling granulator (Model SFC-3 multifunctional granulator manufactured by Fuji Paudal co., ltd). Separately, a liquid in which a predetermined amount of a drug was suspended in a 10 w/w% aqueous solution of hydroxypropylmethylcellulose [HPMC] was added, and this was rolling-granulated. A ratio of blending a drug and a bitter taste masking base was 1:3 as expressed by a weight ratio (solid matter). As a drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used. As an excipient, anhydrous dibasic calcium phosphate (manufactured by Kyowa Chemical Industry Co., Ltd.), and crystalline cellulose (manufacture by Asahi Kasei Corporation) were used. In addition, a viscosity of a HPMC solution at 20°C described in Table 15 is 50 to 14000 mPa·s.

**[Table 15]**

| (weight %) | | |
|---|---|---|
| | Example 26 | Example 27 |
| Isopropylantipyrine | 4.2 | 4.2 |
| HPMC | 12.5 | 12.5 |
| Excipient | Anhydrous dibasic calcium phosphate 83.3 | Crystalline cellulose 83.3 |
| Total | 100.0 | 100.0 |

### (Experimental result)

Experimental results are shown in Table 16. As a result, bitter taste could be masked using a rolling granulator.

**[Table 16]**

| | Example 26 | Example 27 |
|---|---|---|
| Excipient | Anhydrous dibasic calcium phosphate | Crystalline cellulose |
| Masking effect | ○ | ○ |

| | | |
|---|---|---|
| ⊚: Masking effect is very high. ○: Masking effect is high. Δ: Slight masking effect. ×: No masking effect. | | |

### (5) Exclusion granulation process

### (Experimental method)

Dosage form formulation is shown in Table 17. An excipient and a disintegrating agent were placed into a high speed mixer (Model FS2 High Speed Mixer), a liquid in which a predetermined of a drug was suspended in a 20 w/w% aqueous solution of hyroxypropylmethylcellulose [HPMC] was added separately, and this was stirred and granulated. A ratio of blending a drug and a bitter taste masking base was 1:3 as expressed by a weight ratio (solid matter). The granule was extruded with an extrusion granulator (Model DGL1 Dome Gran manufactured by Fuji Paudal co., ltd) to granulate it, and dried with a fluidized bed granulator. As the drug, isopropylantipyrine (manufactured by KONGO CHEMICAL CO., LTD.) was used. As hydroxypropylmethylcellulose which is a bitter taste masking base, hydroxypropylmethylcellulose 2910 (TC-5E, manufactured by Shin-Etsu Chemical Co., Ltd.) was used. As an excipient, anhydrous dibasic calcium phosphate (manufactured by Kyowa Chemical Industry Co., Ltd.) was used. As a disintegrating agent, carmellose calcium (manufactured by Gotoku Chemical Company LTD.) was used. In addition, a viscosity of a HPMC solution at 20°C described in Table 17 is 50 to 14000 mPa·s.

### Stirring granulation conditions

· Granulator: Model FS2 High Speed Mixer
· Production scale: about 150 g
· Mixer rotation number: 300 rpm
· Chopper rotation number: 2500 rpm

### Extrusion granulation conditions

· Granulator: Model DGL1 Dome Gran
· Production scale: about 150 g

### Drying conditions

· Dryer: Model FL-MINI fluidized bed granulator
· Ventilation temperature: 75°C
· Drying completion: Exhaust air temperature 45°C

**[Table 17]**

| (weight %) | |
|---|---|
| | Example 28 |
| Isopropylantipyrine | 4.2 |
| HPMC | 12.5 |
| Disintegrating agent | Carmellose calcium 41.65 |
| Excipient | Anhydrous dibasic calcium phosphate 41.65 |
| Total | 100.0 |

### (Experimental result)

Experimental results are shown in Table 18. As a result, bitter taste could be masked also by using an extrusion granulator.

**[Table 18]**

| | Example 28 |
|---|---|
| Masking effect | ○ |

| | |
|---|---|
| ⊚: Masking effect is very high. ○: Masking effect is high. Δ: Slight masking effect. ×: No masking effect. | |

### (6) Production of dosage form by fluidized bed granulation process after wet grinding

### (Experimental method)

Dosage form formulation is shown in Table 17. An excipient and a disintegrating agent were placed into a high speed mixer (Model FS2 High Speed Mixer), a liquid in which a predetermined amount of a drug was suspended in a 20 w/w% aqueous solution of hydroxypropylmethylcellulose [HPMC] was added separately, and this was stirred and granulated. A ratio of blending a drug and a bitter taste masking base was 1:3 as expressed by a weight ratio (solid matter). The granule was ground with a grinder (Model QC-1975 Comill manufactured by POWLEX CORPORATION), and dried with a fluidized bed granulator. As the drug, isopropylantipyrine (manufactured by KONGO CHEMICAL CO., LTD.) was used. As hydroxypropylmethylcellulose [HPMC] which is a bitter taste masking base, hydroxypropylmethylcellulose 2910 (TC-5E, manufactured by Shin-Etsu Chemical Co., Ltd.) was used. As an excipient, anhydrous dibasic calcium phosphate (manufactured by Kyowa Chemical Industry Co., Ltd.) was used. As a disintegrating agent, carmellose calcium (manufactured by Gotoku Chemical Company LTD.) was used. A viscosity of a HPMC solution at 20°C described in Table 19 is 50 to 14000 mPa·s.

### Stirring granulation conditions

· Granulator: Model FS2 High Speed Mixer
· Production scale: about 150 g
· Mixer rotation number: 300 rpm
· Chopper rotation number: 2500 rpm

### Grinding conditions

· Grinder: Model QC-197S Comill

### Drying conditions

· Dryer: Model FL-MINI fluidized bed granulator
· Ventilation temperature: 75°C
· Drying completion: exhaust air temperature 45°C

**[Table 19]**

| (weight %) | |
|---|---|
| | Example 29 |
| Isopropylantipyrine | 4.2 |
| HPMC | 12.5 |
| Disintegrating agent | Carmellose calcium 41.65 |
| Excipient | Anhydrous dibasic calcium phosphate 41.65 |
| Total | 100.0 |

### (Experimental result)

Experimental results are shown in Table 20. As a result, bitter taste could be masked also when the granule was ground by using a grinder..

**[Table 20]**

| | Example 29 |
|---|---|
| Masking effect | ○ |

| | |
|---|---|
| ⊚: Masking effect is very high. ○: Masking effect is high. Δ: Slight masking effect. ×: No masking effect. | |

### 6. Production of orally disintegrating tablet

### (Experimental method)

Dosage form formulation is shown in Tables 21 and 22. Anhydrous dibasic calcium phosphate, crystalline cellulose, acesulfame potassium, carmellose and magnesium stearate together with granules of Examples 17, and 21 to 25 were bag-mixed, passed through a sieve, and compressed with a single compressing machine (manufactured by Fuji Medical Machine). Compression conditions are as follows.

### Compression conditions

Compressing machine: single compressing machine (Fuji Medical Machine)
Mallet shape: φ6.5 mm two-step R
Compressing pressure: 4.5 to 5.5 kN

**[Table 21]**

| (weight %) | |
|---|---|
| | Examples 30 to 35 |
| Granule of active pharmaceutical ingredient | 24 |
| Anhydrous dibasic calcium phosphate | 38.6 |
| Crystalline cellulose | 26 |
| Acesulfame potassium | 1.4 |
| Carmellose | 10 |
| Magnesium stearate | Minor amount |
| Total | 100 |

**[Table 22]**

| | Examples 30 | Examples 31 | Examples 32 | Examples 33 | Examples 34 | Examples 35 |
|---|---|---|---|---|---|---|
| Granule of active pharmaceutical ingredient | Examples 17 | Examples 21 | Examples 22 | Examples 23 | Examples 24 | Examples 25 |

### (Experimental result)

Results of an organoleptic test are shown in Table 23. As a result, bitter taste could be masked using any excipient or disintegrating agent. Particularly, as in the case of stirring granulation, bitter taste was particularly masked when crospovidone was used as a disintegrating agent.

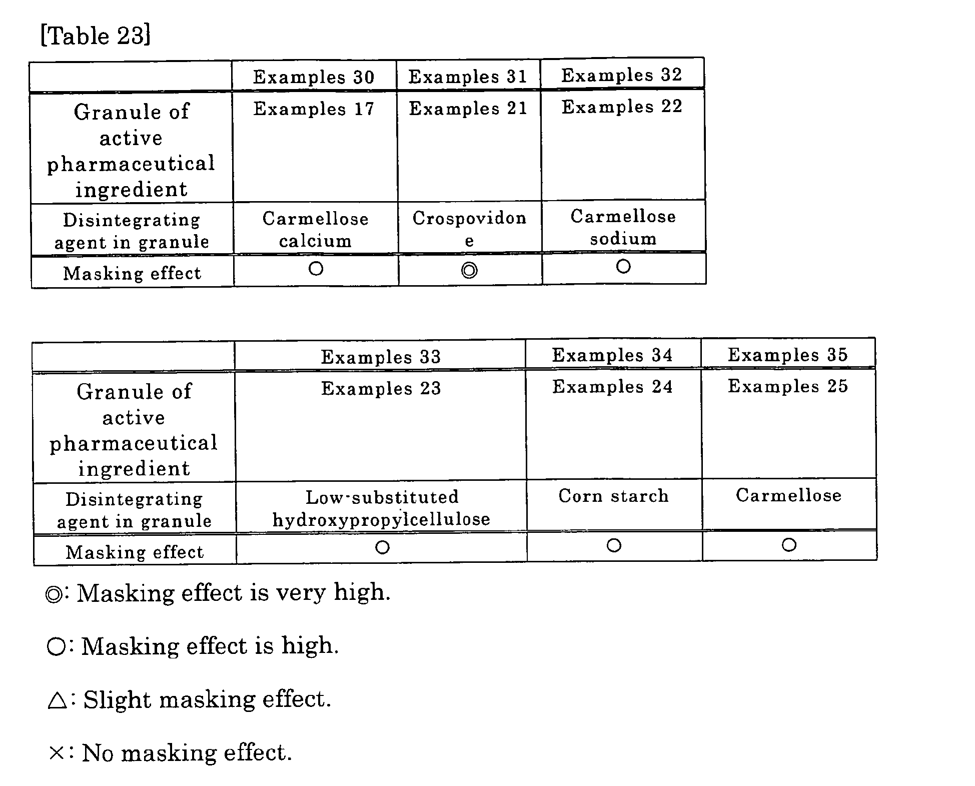

### 7. Production of orally disintegrating tablet containing mint oil

### (Experimental method)

In order to further improve the bitter taste masking effect, a mint oil (manufactured by Shiono Koryo Kaisha, ltd.) was added to a tablet powder of Example 30, and the bitter taste masking effect was confirmed. A mint oil was adsorbed onto hydrated silicon dioxide (manufactured by Degussa), and this was added to a tablet powder.

**[Table 24]**

| (weight %) | | |
|---|---|---|
| | Examples 30 | Examples 36 |
| Granule of active pharmaceutical ingredient | 24 | 24 |
| Anhydrous dibasic calcium phosphate | 38.6 | 38.3 |
| Crystalline cellulose | 26 | 26 |
| Acesulfame potassium | 1.4 | 1.4 |
| Carmellose | 10 | 10 |
| Mint oil | - | 0.1 |
| Hydrated silicon dioxide | - | 0.2 |
| Magnesium stearate | Minor amount | Minor amount |
| Total | 100 | 100 |

### (Experimental result)

When a perfume such as a mint oil was added, the bitter taste masking effect could be further improved.

**[Table 25]**

| | Examples 30 | Examples 36 |
|---|---|---|
| Granule of active pharmaceutical ingredient | Examples 17 | Examples 17 |
| Perfume | None | Mint oil |
| Masking effect | ○ | ⊚ |

| | | |
|---|---|---|
| ⊚: Masking effect is very high. ○: Masking effect is high. Δ: Slight masking effect. ×: No masking effect. | | |

### 8. Production of oral disintegrating tablet containing increased amount of active pharmaceutical ingredient

### (Experimental method)

As shown in Table 26, physical property when an amount of active pharmaceutical ingredient was doubled was assessed. An amount of a active pharmaceutical ingredient granule of Example 17 was doubled, and other additives were almost the same as those of the tablet in Examples 36.

**[Table 26]**

| (weight %) | | |
|---|---|---|
| | Examples 36 | Examples 37 |
| Granule of active pharmaceutical ingredient | 24 | 48 |
| Anhydrous dibasic calcium phosphate | 38.3 | 39.81 |
| Crystalline cellulose | 26 | 27 |
| Acesulfame potassium | 1.4 | 1.8 |
| Carmellose | 10 | 13 |
| Mint oil | 0.1 | 0.13 |
| Hydrated silicon dioxide | 0.2 | 0.26 |
| Magnesium stearate | Minor amount | Minor amount |
| Total | 100 | 100 |

### (Experimental result)

Even when an amount of active pharmaceutical ingredient granule portion was doubled, bitter taste could be masked, and disintegration time and hardness were also good.

**[Table 27]**

| | Examples 36 | Examples 37 |
|---|---|---|
| Masking effect | ⊚ | ○ |
| Disintegration time (sec) | 10 | 14 |
| Hardness (N) | 43.8 | 44.0 |

| | | |
|---|---|---|
| ⊚: Masking effect is very high. ○: Masking effect is high. Δ: Slight masking effect. ×: No masking effect. | | |

### Industrial Applicability

Since the present invention can mask bitter taste only with a base which is dissolved or suspended in water, and only water is used as a solvent, practicability is high. In addition, the present dosage form can be also blended in an intraoral disintegrating tablet, and a tablet which can mask bitter taste while disintegrated in an oral cavity instantly can be produced.

## Claims

1. A process for producing a powder·particle dosage form **characterized in** comprising the following steps:
i) a step of dissolving or suspending a drug having solubility in water of not higher than 1 g/mL (20°C) in a liquid having a viscosity (20°C) having a viscosity of 50 to 14000 mPa·s,
ii) a step of adding the solution or the suspension obtained in the step
i) to an excipient and/or a disintegrating agent, and granulating this.

2. The process for producing a powder·particle dosage form according to claim 1, wherein a viscosity of the liquid (20°C) is 500 to 14000 mPa·s.

3. The process for producing a powder-particle dosage form according to claim 1 or 2, wherein the liquid contains hydroxypropylmethylcellulose.

4. The process for producing a powder·particle dosage form according to claim 1 or 2, wherein the liquid contains fumaric acid, stearic acid and/or polyvinyl acetal diethyl aminoacetate.

5. The process for producing a powder-particle dosage form according to claim 1, wherein the drug is a drug having solubility in water (20°C) of not higher than 0.1 g/mL.

6. The process for producing a powder-particle dosage form according to claim 5, wherein the drug is a drug having solubility in water (20°C) of not higher than 0.033 g/mL.

7. The process for producing a powder-particle dosage form according to any one of claims 1 to 6, wherein the excipient is a water-insoluble excipient.

8. The process for producing a powder·particle dosage form according to any one of claims 1 to 7, wherein the granulation step is a wet granulation process.

9. The process for producing a powder-particle dosage form according to claim 8, wherein the wet granulation process is any of a fluidized bed granulation process, a stirring granulation process, an extrusion granulation process, and a rolling granulation process.

10. The process for producing a powder-particle dosage form according to claim 9, wherein the wet granulation process is a stirring granulation process.

11. A powder-particle dosage form obtained by the process as defined in any one of claims 1 to 10.

12. The powder-particle dosage form according to claim 11, which is a granule.

13. The powder-particle dosage form according to claim 11 or 12, wherein bitter taste is masked.

14. A process for producing a tablet, comprising compressing the powder particle dosage form as defined in claim 11.

15. A process for producing a tablet, comprising mixing the powder· particle dosage form as defined in claim 11 with an excipient, a lubricant, a disintegrating agent and/or a binder, and compressing the mixture.

16. A tablet obtained by the process as defined in claim 14 or 15.

17. The tablet according to claim 16, which is an orally disintegrating tablet.

18. The tablet according to claim 16 or 17, wherein bitter taste is masked.
